(19) Europäisches Patentamt
European Patent Office
Office européen des brevets

(11) **EP 4 076 179 B1**

(12) **EUROPÄISCHE PATENTSCHRIFT**

(45) Veröffentlichungstag und Bekanntmachung des Hinweises auf die Patenterteilung:
**01.11.2023 Patentblatt 2023/44**

(21) Anmeldenummer: **20829186.4**

(22) Anmeldetag: **16.12.2020**

(51) Internationale Patentklassifikation (IPC):
**A61B 5/11** *(2006.01)* **A61B 5/00** *(2006.01)*

(52) Gemeinsame Patentklassifikation (CPC):
**A61B 5/112; A61B 5/1113;** A61B 5/1117;
A61B 5/7275

(86) Internationale Anmeldenummer:
**PCT/AT2020/060472**

(87) Internationale Veröffentlichungsnummer:
**WO 2021/119705 (24.06.2021 Gazette 2021/25)**

(54) **VERFAHREN ZUR BESTIMMUNG DER GANGSICHERHEIT EINER PERSON**

METHOD FOR DETERMINING A PERSON'S GAIT STEADINESS

PROCÉDÉ DE DÉTERMINATION DE LA STABILITÉ DE LA MARCHE D'UNE PERSONNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2019 AT 511482019**

(43) Veröffentlichungstag der Anmeldung:
**26.10.2022 Patentblatt 2022/43**

(73) Patentinhaber: **AIT Austrian Institute of Technology GmbH**
**1210 Wien (AT)**

(72) Erfinder:
• **ZIEGL, Andreas**
**4800 Attnang-Puchheim (AT)**
• **SCHREIER, Günter**
**8010 Graz (AT)**

• **KASTNER, Peter**
**8054 Seiersberg-Pirka (AT)**

(74) Vertreter: **Wildhack & Jellinek**
**Patentanwälte OG**
**Landstraßer Hauptstraße 50**
**1030 Wien (AT)**

(56) Entgegenhaltungen:
**CN-A- 103 720 476    JP-A- 2016 137 226**

• **CHRISTINA STROHRMANN ET AL: "Monitoring motor capacity changes of children during rehabilitation using body-worn sensors",** JOURNAL OF NEUROENGINEERING AND REHABILITATION, BIOMED CENTRAL , LONDON, GB, Bd. 10, Nr. 1, 30. Juli 2013 (2013-07-30), Seite 83, XP021160606, ISSN: 1743-0003, DOI: 10.1186/1743-0003-10-83

**Beschreibung**

Die Erfindung betrifft ein Verfahren zur Bestimmung der Gangsicherheit einer Person, sowie eine Vorrichtung zur Bestimmung der Gangsicherheit einer Person.

Gebrechlichkeit und Stürze sind die Hauptursachen für Morbidität und Behinderung bei älteren Menschen. Rund ein Drittel der Personen über 65 Jahre stürzt mindestens einmal im Jahr. Studien haben gezeigt, dass 40 % der Stürze in Pflegeheimen beim Aufstehen oder Hinsetzen auftreten.

Nach früheren Studien sind die stärksten Risikofaktoren für das Auftreten von Stürzen: frühere Stürze, die körperliche Stärke einer Person, Gleichgewichtsbeeinträchtigungen und der Einsatz bestimmter Medikamente.

Um rechtzeitig vorbeugenden Maßnahmen ergreifen zu können, ist es wichtig, Gangunsicherheiten frühzeitig zu erkennen, und Personen mit erhöhtem Sturzrisiko zu identifizieren, bevor es zu einem ersten Sturz kommt.

Der Timed Up and Go (TUG) Test ist ein häufig verwendetes Werkzeug zur Bewertung des Sturzrisikos älterer Menschen. Dabei wird die Zeit in Sekunden gemessen, die eine Person benötigt um von einem Stuhl aufzustehen, 3 Meter zu gehen, sich umzudrehen, zurück zum Stuhl zu gehen, und sich zu setzen. Der Test umfasst somit eine Vielzahl von funktionalen Mobilitätsaufgaben (TUG-Unteraufgaben), wie z.B. Aufstehen, Gehen, Drehen, Hinsetzen. Dieser Test wird derzeit empfohlen um die Gangsicherheit und das Gleichgewicht zu beurteilen.

Aus dem Stand der Technik sind Vorrichtungen bekannt, die eine standardisierte Durchführung dieses Tests ermöglichen. AT 518810 zeigt beispielsweise eine derartige Vorrichtung, die auch für das erfindungsgemäße Verfahren eingesetzt werden kann.

Das Dokument JP 2016-137226 A beschreibt die Durchführung eines TUG-Tests zum Fallrisiko einer Person, wobei auch ein Modell erwähnt wird. Das Dokument CN 103720476 A beschreibt die Bestimmung einer zurückgelegten Wegstrecke; als Referenz wird ebenfalls ein theoretischer Kreis berechnet. Im Artikel von CHRISTINA STROHRMANN ET AL: "Monitoring motor capacity changes of children during rehabilitation using body-worn sensors",JOURNAL OF NEUROENGINEERING AND REHABILITATION, BIOMED CENTRAL , LONDON, GB, Bd. 10, Nr. 1, Artikel 83 (S. 1-16), 30. Juli 2013, DOI: 10.1186/1743-0003-10-83 wird eine Abweichung vom Weg als möglicher Parameter für einen TUG-Test beschrieben.

Studien, die sich mit der TUG-Zeit befassen, haben jedoch eine begrenzte Aussagekraft der TUG-Zeit bei der Vorhersage von Stürzen gezeigt. Es besteht daher weiterhin das Bedürfnis, ein erhöhtes Sturzrisiko zuverlässig und frühzeitig zu erkennen und die Gangsicherheit einer Person zuverlässiger bestimmen zu können.

Aufgabe der Erfindung ist es daher eine zuverlässige Methode zur Bestimmung der Gangsicherheit bereitzustellen und somit eine frühzeitige Erkennung eines erhöhten Sturzrisikos zu ermöglichen.

Die Erfindung löst diese Aufgabe, indem

- eine Wegstrecke festgelegt wird, wobei die Wegstrecke zumindest zwischen einem Startpunkt und einem Umkehrpunkt verlaufend festgelegt wird und/oder die Wegstrecke zumindest zwischen dem Umkehrpunkt und einem Endpunkt verlaufend festgelegt wird,
- wobei die Bewegung einer Person entlang der Wegstrecke aufgezeichnet wird, wobei eine Zeitnehmung gestartet wird, wenn die Person am Startpunkt oder am Umkehrpunkt die Bewegung entlang der Wegstrecke startet, und wobei die Zeitnehmung gestoppt wird, wenn die Person am Umkehrpunkt oder am Endpunkt die Bewegung entlang der Wegstrecke beendet,
- wobei die Position der Person bezogen auf den Start- und/oder Endpunkt gemessen wird, und wobei die so ermittelten Messwerte einer Verarbeitungseinheit zugeleitet werden, wobei die Verarbeitungseinheit aus den über die Zeit aufgenommenen Messwerten ein Signal erstellt,
- aus dem Verlauf des Signals über die Wegstrecke eine Polynomfunktion als Modell abgeleitet wird und
- der Verlauf des Signals mit dem Modell verglichen wird und aufgrund dieses Vergleichs ein Maßwert für die Gangsicherheit ermittelt wird.

Je genauer das gemessene Signal mit dem abgeleiteten Modell übereinstimmt, desto geringer ist das Sturzrisiko einer Person. Dabei ist das Ergebnis unabhängig davon, ob sich eine Person insgesamt vergleichsweise schnell oder langsam bewegt. Der durch das Verfahren erhaltene Maßwert für die Gangsicherheit ermöglicht daher eine verbesserte Vorhersage des Sturzrisikos.

Vorteilhafte Ausgestaltungen ergeben sich durch die folgenden Merkmale:
Eine besonders einfache Messung wird ermöglicht, wenn die Wegstrecke zwischen dem Startpunkt und dem Umkehrpunkt in einem ersten Teilabschnitt verläuft und/oder wenn die Wegstrecke zwischen dem Umkehrpunkt und dem Endpunkt in einem zweiten Teilabschnitt verläuft, wobei aus dem Verlauf des Signals im ersten und/oder zweiten Teilabschnitt eine Regressionsgerade als Modell abgeleitet wird.

Eine besonders zuverlässige Messung erhält man auch, wenn die Wegstrecke zwischen dem Startpunkt und dem Umkehrpunkt in einem ersten Teilabschnitt verläuft und zwischen dem Umkehrpunkt und dem Endpunkt in einem zweiten Teilabschnitt verläuft, wobei aus dem Verlauf des Signals im ersten und zweiten Teilabschnitt eine Normalverteilung als Modell abgeleitet wird. Dadurch die Wegstrecke in den Teilbereichen mit geringerer Länge festgelegt werden.

Wenn als Maßwert für die Gangsicherheit das Bestimmtheitsmaß $R^2$ verwendet wird, ist eine besonders einfache Interpretation des Ergebnisses möglich. Der erhaltene Wert liegt dabei zwischen 0 und 1, bzw. zwischen 0 % und 100 %. Dieser Wert für die Gangsicherheit kann direkt als Kriterium bei der Evaluierung des Sturzrisikos herangezogen werden.

Das Verfahren liefert besonders zuverlässige und robuste Ergebnisse, wenn die Wegstrecke so festgelegt wird, dass die Person am Umkehrpunkt eine 180° - Drehung ausführt, wobei insbesondere vorgesehen ist, dass der erste und zweite Teilabschnitt entlang einer, insbesondere derselben, Geraden verläuft, und/oder dass der Startpunkt und der Endpunkt den gleichen Abstand zum Umkehrpunkt aufweisen, vorzugsweise identisch sind.

Das Verfahren kann im Rahmen eines standardisierten TUG-Testes angewendet werden, bzw. können im Rahmen dieses Tests erhaltenen Daten für das Verfahren herangezogen werden. Eine standardisierte und daher besonders robuste Bestimmung der Gangsicherheit kann erfolgen, wenn

- ein Stuhl am Startpunkt positioniert wird,
- ein Entfernungsmessgerät an der Rückenlehne des Stuhl befestigt wird, wobei das Entfernungsmessgerät den Abstand einer vor einem Stuhl befindlichen Testperson von der Rückenlehne des Stuhls laufend misst,
- wobei die vom Entfernungsmessgerät aufgezeichneten Entfernungsmesswerte laufend aufgezeichnet werden,
- wobei die Testperson angewiesen wird, vom Stuhl aufzustehen, vorwärts zum Umkehrpunkt zu gehen, am Umkehrpunkt umzukehren, vorwärts zum Endpunkt zurückzugehen und sich wieder auf dem Stuhl hinzusetzen, und diese Anweisung nach ihren Möglichkeiten auszuführen,
- wobei überprüft wird, ob die Testperson auf dem Stuhl sitzt und wenn dies für eine vorgegebene Zeitspanne der Fall ist, die Zeitnehmung gestartet wird, wobei insbesondere ein vorgegebenes Start-Signal abgegeben wird, und
- wobei die Zeitnehmung gestoppt wird, wenn sich die Testperson wieder auf den Stuhl setzt.

Um die Gangsicherheit einer Person besonders einfach bestimmen zu können, kann vorgesehen sein, dass das Signal, insbesondere nach dem Ende der Zeitnehmung, daraufhin untersucht wird, ob das Signal

a) in einem ersten Zeitbereich ansteigt,
b) nach dem Übersteigen eines vorgegebenen Umkehrschwellenwerts wieder abnimmt, bis es einen unteren Schwellenwert unterschreitet, und

sofern die Vorraussetzungen a) und b) vorliegen, das Verfahren als korrekt durchgeführt angesehen wird.

Es wird daher kein Testleiter benötigt, der die korrekte Durchführung des Tests bestätigt. Eine weitere Möglichkeit eine Durchführung des Tests ohne Testleiter zu ermöglichen besteht darin, dass der Maßwert als inkorrekt angesehen wird,

- wenn die Steigung des Signals einen vorgegebenen Steigungsschwellenwert überschreitet und/oder
- wenn das Signal Messwerte aufweist, die die Entfernung des Startpunkts zum Umkehrpunkt um einen vorgegebenen Schwellenwert übersteigen, und/oder
- wenn keiner der Entfernungsmesswerte des Signals einen vorgegebenen Schwellenwert erreicht und die Testperson den Umkehrpunkt nicht erreicht, und/oder
- wenn die Zeitnehmung eine Maximalzeit erreicht, ohne dass der Endpunkt erreicht wurde.

Eine besonders zuverlässige Möglichkeit der Messung wird erhalten, wenn das Entfernungsmessgerät die Entfernung mittels Ultraschall- oder Lasermessung bestimmt. Um das Verfahren besonders flexibel einsetzen zu können, ist erfindungsgemäß auch ein Datenträger, auf dem ein Computerprogramm zur Durchführung der im zuvor beschriebenen Verfahren von der Verarbeitungseinheit ausgeführten Schritte abgespeichert ist.

Um gerade ältere Personen vor der Gefahr einer dauerhaften Beeinträchtigung zu schützen, ist es notwendig, das Sturzrisiko mit möglichst geringem Aufwand zuverlässig bestimmen zu können.

Aufgabe der Erfindung ist daher weiters eine Vorrichtung zur Bestimmung der Gangsicherheit bereitzustellen, die eine einfache Handhabung ermöglicht.

Diese Aufgabe wird gelöst durch eine Vorrichtung umfassend

- zumindest einen Sensor zur Zeitmessung,
- zumindest einen Sensor zur Aufzeichnung der Position einer Person, die sich in einem bestimmten Bereich befindet,
- eine Verarbeitungseinheit, wobei die Verarbeitungseinheit dazu ausgebildet ist, aus den über die Zeit aufgenommenen Messwerten ein Signal zu erstellen, aus dem Verlauf des Signals ein Modell abzuleiten und den Verlauf des Signals mit dem Modell zu vergleichen und aufgrund dieses Vergleichs einen Maßwert für die Gangsicherheit abzuleiten.

Durch eine derartige Vorrichtung ist eine unauffällige und zuverlässige Messung der Gangsicherheit im Alltag möglich.

Eine besonders zuverlässige Bestimmung der Gangsicherheit kann mit einem standardisierten Versuchsaufbau erfolgen, wobei die Vorrichtung umfasst:

- einen Stuhl mit einer Sitzfläche und einer Rückenlehne,

    - eine mit dem Stuhl verbundene Verarbeitungseinheit,
    - ein an die Verarbeitungseinheit angeschlossenes und mit der Rückenlehne verbundenes Entfernungsmessgerät, das den Abstand einer vor einem Stuhl befindlichen Testperson von der Rückenlehne des Stuhls laufend misst, und
    - ein der Verarbeitungseinheit nachgeschalteter Signalgeber, der ausgebildet ist, bei Vorliegen eines Trigger-Signals oder bei Feststellung, dass eine Person auf dem Stuhl sitzt, ein Startsignal abzugeben,
    - eine Zeitgebung, die durch das Startsignal aktivierbar ist,

    wobei die Steuereinheit dazu ausgebildet ist, nach dem Ende der Zeitnehmung, aus den über die Zeit aufgenommenen Messwerten ein Signal zu erstellen, und
- aus dem Verlauf des Signals über die gesamte Wegstrecke eine Normalverteilung als Modell abzuleiten, und/oder aus dem Verlauf des Signals eine Regressionsgerade als Modell abzuleiten, und
- den Verlauf des Signals mit dem Modell zu vergleichen und das Bestimmtheitsmaß R² als Maßwert für die Gangsicherheit bereitzuhalten.

Ein Ausführungsform eines erfindungsgemäßen Verfahrens ist anhand der folgenden Figuren ohne Einschränkung des durch die Ansprüche definierten Schutzumfangs beispielhaft dargestellt:

Fig. 1 zeigt das Signal eines TUG-Test und die Anpassung an eine Normalverteilung.
Fig. 2 zeigt das Signal eines TUG-Tests und die Anpassung an eine lineare Regression.
Fig. 3 zeigt das Bestimmtheitsmaß R² für die Modelle aus Fig. 1 und Fig. 2.
Fig. 4 zeigt die TUG-Zeit und TUG-Zeit für Unteraufgaben bei gestürzten und nicht gestürzten Personen.
Fig. 5 zeigt die Operationscharakteristik für das Normalverteilungs-Modell, das lineare Regressions-Modell, die Situptime und die TUG-Time.

Beispiel:

39 Personen im Alter von ≥ 65 Jahren wurden über einen Zeitraum von 13 Monaten beobachtet, wobei innerhalb von 15 Wochen jeweils 6 TUG-Messungen mit ähnlichen zeitlichen Abständen durchgeführt. Zusätzlich wurden alle Stürze der Teilnehmer erfasst. Eine Person wurde als "gestürzt" klassifiziert, wenn die Person im Beobachtungszeitraum mindestens einmal gestürzt ist. Während des Beobachtungszeitraums stürzten 23 Teilnehmer und es wurden insgesamt 89 Stürze beobachtet, 16 Teilnehmer blieben ohne Sturz.

Die TUG-Messungen wurden mit eine Ultraschall-TUG-Vorrichtung durchgeführt, wie sie in AT518810 offenbart ist. Diese Vorrichtung maß einerseits die Zeit in Sekunden, die die Testperson zur Durchführung eines TUG-Tests benötigte, weiters wurde von einem Entfernungsmessgerät durch einen Ultraschall-Sensor, mit einer Pulsrate von 10 Hz, kontinuierlich der Abstand der Testperson zum Stuhl bestimmt und derart die Bewegung der Testperson entlang einer Test-Wegstrecke aufgezeichnet. Das Entfernungsmessgerät wurde an der Rückenlehne eines Stuhls befestigt und der Stuhl mit dem Gerät wurde 3,5 Meter vor eine Wand als Umkehrpunkt gestellt. Nachdem sich die Testperson auf den Stuhl setzte, wurde ein Startsignal gegeben und die Zeitnehmung gestartet. Die Testperson stand daraufhin auf, ging gerade auf die Wand zu, drehte sich direkt vor der Wand um 180° um, ging vorwärts auf direktem Weg zurück zum Stuhl und setzte sich. Nach dem Hinsetzen stoppte die Vorrichtung die Aufnahme und wertete den Test aus. Zum Vergleich der Ergebnisse von Personen, die als gestürzt klassifiziert wurden, und Personen, die nicht als gestürzt klassifiziert wurden, wurden zweiseitig ungepaarte t-Tests (p<0,05) verwendet.

Die Signale wurden vor der Auswertung von Spikes bereinigt, wobei als Spikes alle Bereiche gewertet wurden, die eine Beschleunigung von > 4 m/s² aufwiesen. Die Distanzmessung wurde weiters herangezogen, um die für die TUG-Unteraufgaben benötigte Zeit zu erheben.

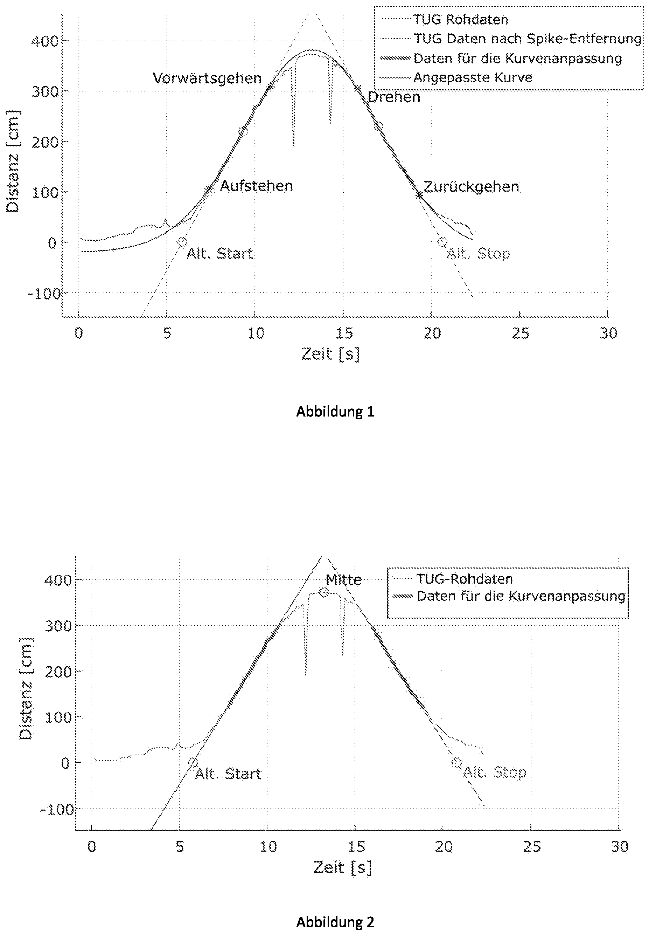

**Fig. 1** zeigt die Signalauswertung, wobei zum einen die Rohdaten dargestellt sind, weiters das Signal nach Entfernung der Spikes, der zur Auswertung herangezogene Bereich des Signals und die als Modell abgeleitete Gaußsche Kurve. Die Gaußsche Kurve wurde für den Bereich zwischen dem Aufstehen bis 50 cm vor dem Umkehrpunkt und 50 cm nach dem Umkehrpunkt bis zum Hinsetzen angepasst.

Das Model wurde durch Anpassung der Koeffizienten erhalten, wobei für

$$y(x) = a + be^{\left[-\left(\frac{x-c}{d}\right)^2\right]}$$

a, b, c und d die Koeffizienten der Approximation der Binomialverteilung darstellen. Das Bestimmtheitsmaß R² wurde wie folgt berechnet:

$$R^2 = \frac{SSR}{SST} = 1 - \frac{SSE}{SST}$$

SSR ist dabei die Residuenquadratsumme, SST ist die totale Quadratsumme und SSE steht für die erklärte Quadratsumme.

Fig. 2 zeigt das Signal das im Verlauf eines TUG-Tests aufgenommen wurde und die Anpassung an ein lineares Regressions-Modell. Ein alternativer Startpunkt und ein alternativer Endpunkt wurden berechnet, indem der Schnittpunkt der Kurventangente mit der größten Steigung mit der x-Achse berechnet wurde.

Dabei wurde je eine Regressionsgerade mit positiver und negativer Steigung an beide Flanken des Signalverlaufs angepasst. y1 gibt die Regressionsgerade für die ansteigende Flanke wieder, y2, die Regressionsgerade für die abfallende Flanke.

$$y_1(x) = \beta_0 + x\beta_1$$

$$y_2(x) = \beta_2 + x\beta_3$$

Die Schnittpunkte der angepassten Regressionsgeraden mit der x-Achse wurden als alternativer Startpunkt und alternativer Endpunkt bezeichnet. Dazu wurden die Koeffizienten $\beta_0$, $\beta_1$ $\beta_2$ und $\beta_3$ für die Berechnung herangezogen.

$$Alt.start = -\frac{\beta_0}{\beta_1}$$

$$Alt.stop = -\frac{\beta_2}{\beta_3}$$

Als Zentrum wurde der Mittelwert zwischen alternativem Startpunkt und alternativem Endpunkt berechnet. Die Zeit, die eine Testperson zur Durchführung benötigte, wurde berechnet, indem der Wert am alternativen Startpunkt vom Wert am alternativen Endpunkt abgezogen wurde.

Weiters wurden folgende Parameter erhoben: mittlere quadratische Abweichung, Bestimmtheitsmaß, Geschwindigkeit auf dem ersten Teilabschnitt, Geschwindigkeit auf dem zweiten Teilabschnitt und Unterschied zwischen der positiven und der negativen Steigung der jeweiligen Regressionsgeraden.

Anschließend wurde ausgewertet, welche Parameter eine zuverlässige Vorhersage erlauben, ob eine Person als "gestürzt" oder "nicht gestürzt" klassifiziert wurde.

Die Ergebnisse wurden als Boxplot-Diagramme dargestellt.

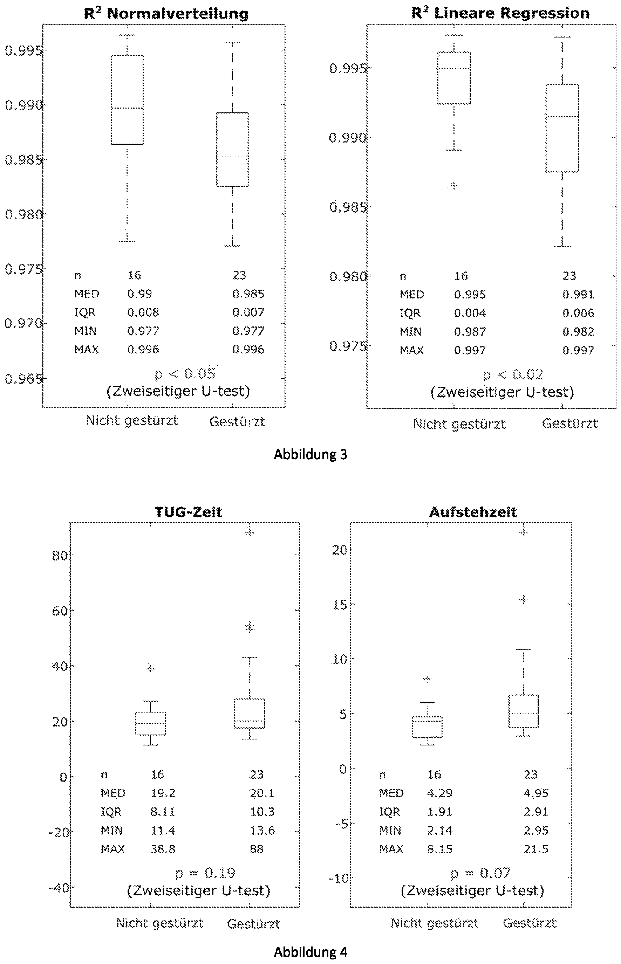

**Fig. 3** zeigt die Verteilung der Werte für das Bestimmtheitsmaß $R^2$. Dabei konnten signifikante Unterschiede zwischen "gestürzten" und "nicht-gestürzten" Personen festgestellt werden.

Für das Normalverteilungs-Modell lag der p-Wert < 0,05 erhalten. Bei den 16 Personen, die als "nicht-gestürzt" klassifiziert wurden, lag der Mittelwert für $R^2$ bei 0,990, das Minimum lag bei 0,977, das Maximum bei 0,996. Dabei trat ein Quartilsabstand von 0,00811 auf. Bei den 23 als "gestürzt" klassifizierten Personen lag der Mittelwert für $R^2$ bei 0,985, und der Quartilsabstand bei 0,00626.

Für das lineare Regressions-Modell wurde ein p-Wert < 0,02 erhalten. In diesem Fall lag der Mittelwert bei "gestürzten" Personen bei 0,991, bei "nicht-gestürzten" Personen bei 0,995. Das Minimum wurde für "nicht-gestürzte" Personen bei 0,987 erreicht, das Maximum bei 0,997, der Quartilsabstand lag bei 0,00371. Bei "gestürzten" Personen zeigte sich das Minimum bei 0,982, das Maximum bei 0,997 und der Quartilsabstand wurde mit 0,00626 festgestellt.

Es hat sich somit gezeigt, dass beide abgeleiteten Modelle eine zuverlässige Vorhersage der Klassifikation der teilnehmenden Personen in "gestürzt" und "nicht-gestürzt" erlaubten.

**Fig. 4** zeigt als Vergleich die Auswertung der gesamten TUG-Zeit und der Situptime, also der Zeit, die zum Aufstehen benötigt wurde, jeweils differenziert nach "gestürzten" und "nicht-gestürzten" Personen.

Für die gesamte TUG-Zeit war für die "nicht-gestürzten" Personen der Mittelwert 19,2 Sekunden, das Minimum war 11,4 Sekunden, das Maximum lag bei 38,8 Sekunden, der Quartilsabstand war 8,11. Bei den als "gestürzt" klassifizierten Personen lag der Mittelwert bei 20,1 Sekunden, das Minimum bei 13,6 Sekunden und das Maximum bei 88 Sekunden. Der Quartalsabstand lag bei 10,3.

Bei der Situptime war für die "nicht-gestürzten" Personen der Mittelwert 4,29 Sekunden, das Minimum war 2,14 Sekunden und das Maximum 8,15 Sekunden. Der Quartilsabstand lag bei 1,91. Bei den "gestürzten" Personen lag der Mittelwert bei 4,95 Sekunden, das Minimum bei 2,95 Sekunden, das Maximum bei 21,5 Sekunden und der Quartilsabstand bei 2,91.

Für beide Parameter war die benötigte Zeit bei "gestürzten" Personen somit tendenziell länger als bei "nicht-gestürzten" Personen. Allerdings ergaben sich keine signifikanten Unterschiede (TUG-Zeit: p< 0,19; Aufstehzeit: p < 0,07).

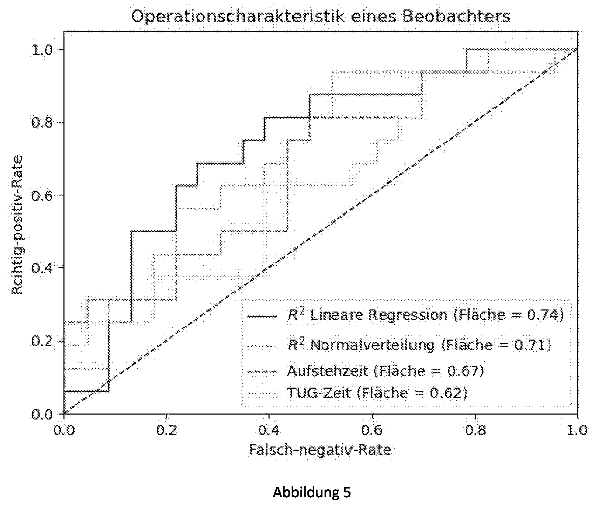

**Fig. 5** zeigt die Operationscharakteristik, wobei für alle vier Modelle die Eignung zur Klassifikation untersucht wurde. Dazu wurde die Tatsächlich-positiv-Rate (TP) und die Falsch-positiv-Rate (FP) bewertet. Der Bereich innerhalb der Kurve, auch "Bereich unter der Kurve" (AUC) genannt, kann zur Beurteilung der Zuverlässigkeit der Modelle herangezogen werden. Die AUC für das lineare Regressions-Modell war 0,74, für das Normalverteilungs-Modell 0,71. Die AUC der Vergleichswerte waren 0,62 für die gesamte TUG-Zeit und 0,67 für die Situptime.

Es zeigte sich somit, dass das Normalverteilungs-Modell und das lineare Regressions-Modell eine zuverlässige Vorhersage erlaubten, ob eine Person als "gestürzt" oder "nicht-gestürzt" klassifiziert wurde.

Diese Modelle erlauben es daher einen Maßwert für die Gangsicherheit abzuleiten, der zur Feststellung eines erhöhten Sturzrisikos herangezogen werden kann. Damit können Personen mit erhöhtem Sturzrisiko frühzeitiger und zuverlässiger festgestellt werden und geeignete Maßnahmen zur Verhinderung einer sturzbedingten Verletzung können rechtzeitig getroffen werden.

**Patentansprüche**

1. Verfahren zur Bestimmung der Gangsicherheit einer Person, wobei

    - eine Wegstrecke festgelegt wird, wobei die Wegstrecke zumindest zwischen einem Startpunkt und einem Umkehrpunkt verlaufend festgelegt wird und/oder die Wegstrecke zumindest zwischen dem Umkehrpunkt und einem Endpunkt verlaufend festgelegt wird,
    - die Bewegung einer Person entlang der Wegstrecke aufgezeichnet wird, wobei eine Zeitnehmung gestartet wird, wenn die Person am Startpunkt oder am Umkehrpunkt die Bewegung entlang der Wegstrecke startet, und wobei die Zeitnehmung gestoppt wird, wenn die Person am Umkehrpunkt oder am Endpunkt die Bewegung entlang der Wegstrecke beendet,
    - die Position der Person bezogen auf den Start- und/oder Endpunkt gemessen wird, und wobei die so ermittelten Messwerte einer Verarbeitungseinheit zugeleitet werden, wobei die Verarbeitungseinheit aus den über die Zeit aufgenommenen Messwerten ein Signal erstellt,
    - aus dem Verlauf des Signals über die Wegstrecke eine Polynomfunktion als Modell abgeleitet wird,
    **dadurch gekennzeichnet, dass**
    - der Verlauf des Signals mit dem Modell verglichen wird und aufgrund dieses Vergleichs ein Maßwert für die Gangsicherheit ermittelt wird.

2. Verfahren nach Anspruch 1, wobei die Wegstrecke zwischen dem Startpunkt und dem Umkehrpunkt in einem ersten Teilabschnitt verläuft und/oder wobei die Wegstrecke zwischen dem Umkehrpunkt und dem Endpunkt in einem zweiten Teilabschnitt verläuft, wobei aus dem Verlauf des Signals im ersten und/oder zweiten Teilabschnitt eine Regressionsgerade als Modell abgeleitet wird.

3. Verfahren nach Anspruch 1, wobei die Wegstrecke zwischen dem Startpunkt und dem Umkehrpunkt in einem ersten Teilabschnitt verläuft und zwischen dem Umkehrpunkt und dem Endpunkt in einem zweiten Teilabschnitt verläuft, wobei aus dem Verlauf des Signals im ersten und zweiten Teilabschnitt eine Normalverteilung als Modell abgeleitet wird.

4. Verfahren nach einem der Ansprüche 1 bis 3, wobei als Maßwert das Bestimmtheitsmaß $R^2$ abgeleitet wird.

5. Verfahren nach einem der Ansprüche 1 bis 4, wobei die Wegstrecke so festgelegt wird, dass die Person am Umkehrpunkt eine 180°Drehung ausführt, wobei insbesondere vorgesehen ist, - dass der erste und zweite Teilabschnitt entlang einer, insbesondere derselben, Geraden verläuft, und/oder

    - dass der Startpunkt und der Endpunkt den gleichen Abstand zum Umkehrpunkt aufweisen, vorzugsweise identisch sind.

6. Verfahren nach einem der Ansprüche 1 bis 5,

    - wobei ein Stuhl am Startpunkt positioniert wird,
    - wobei an der Rückenlehne des Stuhl ein Entfernungsmessgerät angebracht wird, das den Abstand einer vor einem Stuhl befindlichen Testperson von der Rückenlehne des Stuhls laufend misst,
    - wobei die vom Entfernungsmessgerät aufgezeichneten Entfernungsmesswerte laufend aufgezeichnet werden,
    - wobei die Testperson angewiesen wird, vom Stuhl aufzustehen, vorwärts zum Umkehrpunkt zu gehen, am Umkehrpunkt umzukehren, zum Endpunkt zurückzugehen und sich wieder auf dem Stuhl hinzusetzen, und diese Anweisung bestmöglich auszuführen,
    - wobei überprüft wird, ob die Testperson auf dem Stuhl sitzt und wenn dies für eine vorgegebene Zeitspanne der Fall ist, die Zeitnehmung gestartet wird und wobei insbesondere vorgesehen ist, dass ein vorgegebenes Start-Signal abgegeben wird,
    - wobei die Zeitnehmung gestoppt wird, wenn sich die Testperson wieder auf den Stuhl setzt.

7. Verfahren nach einem der Ansprüche 1 bis 6, wobei das Signal, insbesondere nach dem Ende der Zeitnehmung, daraufhin untersucht wird, ob das Signal

    a) in einem ersten Zeitbereich ansteigt,
    b) nach dem Übersteigen eines vorgegebenen Umkehrschwellenwerts wieder abnimmt, bis es einen unteren Schwellenwert unterschreitet, und

    sofern die Vorraussetzungen a) und b) vorliegen, der Maßwert als korrekt angesehen wird.

**8.** Verfahren nach einem der Ansprüche 1 bis 7, wobei der Maßwert als inkorrekt angesehen wird,

- wenn die Steigung des Signals einen vorgegebenen Steigungsschwellenwert überschreitet und/oder
- wenn das Signal Messwerte aufweist, die die Entfernung des Startpunkts zum Umkehrpunkt um einen vorgegebenen Schwellenwert übersteigen, und/oder
- wenn keiner der Entfernungsmesswerte des Signals einen vorgegebenen Schwellenwert erreicht und die Testperson den Umkehrpunkt nicht erreicht, und/oder
- wenn die Zeitnehmung eine Maximalzeit erreicht, ohne dass der Endpunkt erreicht wurde.

**9.** Verfahren nach einem der Ansprüche 1 bis 8, wobei das Entfernungsmessgerät die Entfernung mittels Ultraschall- oder Lasermessung bestimmt.

**10.** Datenträger, umfassend ein Computerprogramm, das bei der Ausführung durch einen Computer, diesen veranlasst

- Messwerte, die die Bewegung einer Person entlang einer Wegstrecke wiedergeben, zu empfangen,
- aus den über die Zeit aufgenommenen Messwerten ein Signal zu erstellen,
- aus dem Verlauf des Signals über die Wegstrecke eine Polynomfunktion als Modell abzuleiten,
**dadurch gekennzeichnet, dass** das Computerprogramm den Computer bei Ausführung außerdem veranlasst,
- den Verlauf des Signals mit dem Modell zu vergleichen und aufgrund dieses Vergleichs einen Maßwert für die Gangsicherheit zu ermitteln.

**11.** Vorrichtung zur Bestimmung der Gangsicherheit, umfassend

- zumindest einen Sensor zur Zeitmessung,
- zumindest einen Sensor zur Aufzeichnung der Position einer Person, die sich auf einer zuvor festgelegten Wegstrecke befindet,
- eine Verarbeitungseinheit, wobei die Verarbeitungseinheit dazu ausgebildet ist, aus den über die Zeit aufgenommenen Messwerten ein Signal zu erstellen, aus dem Verlauf des Signals ein Modell abzuleiten,

**dadurch gekennzeichnet, dass** die Auswerteeinheit außerdem dazu ausgebildet ist, den Verlauf des Signals mit dem Modell zu vergleichen und aufgrund dieses Vergleichs einen Maßwert für die Gangsicherheit abzuleiten.

**12.** Vorrichtung nach Anspruch 11, wobei die Vorrichtung umfasst:

- einen Stuhl mit einer Sitzfläche und einer Rückenlehne,

- eine mit dem Stuhl verbundene Verarbeitungseinheit,
- ein an die Verarbeitungseinheit angeschlossenes und mit der Rückenlehne verbundenes Entfernungsmessgerät, das den Abstand einer vor einem Stuhl befindlichen Person von der Rückenlehne des Stuhls laufend misst, und
- ein der Verarbeitungseinheit nachgeschalteter Signalgeber, der ausgebildet ist, bei Vorliegen eines Trigger-Signals oder bei Feststellung, dass eine Person auf dem Stuhl sitzt, ein Startsignal abzugeben,
- eine Zeitgebung, die durch das Startsignal aktivierbar ist,

wobei die Steuereinheit dazu ausgebildet ist, nach dem Ende der Zeitnehmung, aus den über die Zeit aufgenommenen Messwerten ein Signal zu erstellen, und
- aus dem Verlauf des Signals eine Polynomfunktion abzuleiten und
- den Verlauf des Signals mit dem Modell zu vergleichen und einen Maßwert für die Gangsicherheit, insbesondere das Bestimmtheitsmaß $R^2$, bereitzuhalten.

**Claims**

**1.** A method for determining the walking safety of a person, wherein

- a path distance is determined, wherein the path distance is determined to run at least between a starting point and a turning point and/or the path distance is determined to run at least between the turning point and an end point,
- the movement of a person along the path distance is recorded, wherein a timing is started when the person starts the movement along the path at the starting point or at the turning point, and wherein the timing is stopped when the person stops the movement along the path at the turning point or at the end point,
- the position of the person relative to the starting point and/or end point is measured, and wherein the measured values determined in this way are fed to a processing unit, wherein the processing unit generates a signal from the measured values recorded over time,

- a polynomial function is derived as a model from the progression of the signal over the path distance,

**characterised in that**

- the progression of the signal is compared to the model and a measured value for the walking safety is determined on the basis of this comparison.

2. The method according to claim 1, wherein the path distance between the starting point and the turning point runs in a first subsection and/or wherein the path distance between the turning point and the end point runs in a second subsection, wherein a regression line is derived as a model from the progression of the signal in the first and/or second subsection.

3. The method according to claim 1, wherein the path distance between the starting point and the turning point runs in a first subsection and between the turning point and the end point runs in a second subsection, wherein a normal distribution is derived as a model from the progression of the signal in the first and second subsections.

4. The method according to any one of claims 1 to 3, wherein the coefficient of determination $R^2$ is derived as the measured value.

5. The method according to any one of claims 1 to 4, wherein the path distance is determined in such a way that the person executes a 180° turn at the turning point, wherein in particular it is provided

- that the first and second subsection run along one, in particular the same, straight line, and/or
- that the starting point and the end point have the same distance to the turning point, preferably are identical.

6. The method according to any of claims 1 to 5,

- wherein a chair is positioned at the starting point,
- wherein a distance measuring device is attached to the backrest of the chair, the distance measuring device continuously measuring the distance of a test person located in front of a chair from the backrest of the chair,
- wherein the distance measurement values recorded by the distance measurement device are continuously recorded,
- wherein the test person is instructed to stand up from the chair, walk forward to the turning point, turn around at the turning point, walk back to the end point, and sit back down on the chair, and to carry out this instruction to the best of their ability,

- wherein it is checked whether the test person is sitting on the chair and if this is the case for a predetermined period of time, the timing is started and wherein it is provided in particular that a predetermined start signal is emitted,
- whereby the timing is stopped when the test person sits down on the chair again.

7. The method according to any of claims 1 to 6, wherein the signal, in particular after the end of the timing, is examined to determine whether the signal

a) increases in a first time range,
b) decreases again after exceeding a predetermined turning threshold until it falls below a lower threshold, and

if conditions a) and b) are fulfilled, the measured value is considered to be correct.

8. The method according to any of claims 1 to 7, wherein the measured value is regarded as incorrect,

- if the slope of the signal exceeds a predetermined slope threshold value and/or
- if the signal has measurement values that exceed the distance from the starting point to the turning point by a predetermined threshold value, and/or
- if none of the distance measurement values of the signal reaches a predetermined threshold and the test person does not reach the turning point, and/or
- when the timing reaches a maximum time without reaching the end point.

9. The method according to any one of claims 1 to 8, wherein the distance measuring device determines the distance using ultrasonic or laser measurement.

10. A data carrier comprising a computer program which, when executed by a computer, causes the computer to

- receive measured values representing the movement of a person along a path distance,
- create a signal from the measured values recorded over time,
- derive a polynomial function as a model from the progression of the signal over the path distance,

**characterized in that** the computer program, when executed, also causes the computer to

- compare the progression of the signal with the model and determine a measured value for walking safety on the basis of this comparison.

**11.** A device for determining walking safety, comprising

- at least one sensor for the time measurement,
- at least one sensor for recording the position of a person who is on a previously determined path distance,
- a processing unit, wherein the processing unit is designed to generate a signal from the measured values recorded over time, to derive a model from the progression of the signal,

**characterized in that** the evaluation unit is further configured to compare the progression of the signal with the model and to derive a measured value for the walking safety on the basis of this comparison.

**12.** The device according to claim 11, wherein the device comprises:

- a chair having a seat and a backrest,

- a processing unit connected to the chair,
- a distance measuring device connected to the processing unit and connected to the backrest, the distance measuring device continuously measuring the distance of a person located in front of a chair from the backrest of the chair, and
- a signal generator connected downstream of the processing unit, the signal generator being configured to emit a start signal when a trigger signal is present or when it is detected that a person is sitting on the chair,
- a timing unit which can be activated by the start signal,

wherein the control unit is configured to generate a signal after the end of the timing from the measured values recorded over time, and
- to derive a polynomial function from the progression of the signal, and
- to compare the progression of the signal with the model and to hold ready a measured value for the walking safety, in particular the coefficient of determination R2.

**Revendications**

**1.** Procédé pour déterminer la sécurité de déplacement d'une personne, dans lequel

- un trajet est défini, dans lequel le trajet est défini comme s'étendant au moins entre un point de départ et un point d'inversion et/ou le trajet est défini comme s'étendant au moins entre le point d'inversion et un point final,
- le mouvement d'une personne le long du trajet est enregistré, dans lequel un chronométrage est lancé lorsque la personne commence le mouvement le long du trajet au point de départ ou au point d'inversion, et dans lequel le chronométrage est arrêté lorsque la personne termine le mouvement le long du trajet au point d'inversion ou au point final,
- la position de la personne est mesurée par rapport au point de départ et/ou au point d'arrivée, et dans lequel les valeurs de mesure ainsi déterminées sont transmises à une unité de traitement, l'unité de traitement établissant un signal à partir des valeurs de mesure enregistrées au fil du temps,
- une fonction polynomiale est déduite comme modèle à partir de l'évolution du signal sur la distance,

**caractérisé en ce que**
- l'évolution du signal est comparée au modèle et une valeur de mesure pour la sécurité de marche est déterminée sur la base de cette comparaison.

**2.** Procédé selon la revendication 1, dans lequel le trajet entre le point de départ et le point d'inversion s'étend dans un premier tronçon partiel et/ou dans lequel le trajet entre le point d'inversion et le point final s'étend dans un second tronçon partiel, dans lequel une droite de régression est dérivée comme modèle à partir de l'évolution du signal dans le premier et/ou le second tronçon partiel.

**3.** Procédé selon la revendication 1, dans lequel le trajet entre le point de départ et le point d'inversion s'étend dans un premier sous-tronçon et entre le point d'inversion et le point final s'étend dans un second sous-tronçon, dans lequel une distribution normale est dérivée comme modèle à partir de l'évolution du signal dans le premier et le second sous-tronçons.

**4.** Procédé selon l'une quelconque des revendications 1 à 3, dans lequel le coefficient de détermination R2 est dérivé en tant que valeur de mesure.

**5.** Procédé selon l'une quelconque des revendications 1 à 4, dans lequel le trajet est déterminé de telle sorte que la personne effectue une rotation de 180° au point d'inversion, dans lequel il est notamment prévu :

- que le premier et le second tronçons s'étendent le long d'une droite, notamment identique, et/ou
- que le point de départ et le point d'arrivée présentent la même distance par rapport au point d'inversion, et sont de préférence identiques.

**6.** Procédé selon l'une quelconque des revendications 1 à 5,

> - dans lequel une chaise est positionnée au point de départ,
> - dans lequel un télémètre est monté sur le dossier de la chaise, lequel mesure en continu la distance entre un sujet se trouvant devant une chaise et le dossier de la chaise,
> - dans lequel les valeurs de mesure de distance enregistrées par le télémètre sont enregistrées en continu,
> - dans lequel il est demandé au sujet de se lever de la chaise, d'avancer jusqu'au point d'inversion, de faire demi-tour au point d'inversion, de revenir au point final et de s'asseoir à nouveau sur la chaise, et d'exécuter cette consigne de la meilleure manière possible,
> - dans lequel il est vérifié que la personne testée est assise sur la chaise et si tel est le cas pendant un laps de temps prédéfini, le chronométrage est démarré et dans lequel il est notamment prévu qu'un signal de démarrage prédéfini soit émis,
> - dans lequel le chronométrage s'arrête lorsque la personne testée s'assoit à nouveau sur la chaise.

**7.** Procédé selon l'une quelconque des revendications 1 à 6, dans lequel le signal est analysé, en particulier après la fin du chronométrage, pour déterminer si le signal

> a) augmente dans une première plage de temps,
> b) diminue à nouveau après avoir dépassé un seuil d'inversion prédéterminé jusqu'à ce qu'elle passe en dessous d'un seuil inférieur, et

dans la mesure où les conditions a) et b) sont remplies, la valeur de mesure est considérée comme correcte.

**8.** Procédé selon l'une quelconque des revendications 1 à 7, dans lequel la valeur de mesure est considérée comme incorrecte,

> - si la pente du signal dépasse un seuil de pente prédéfini et/ou
> - si le signal présente des valeurs mesurées qui dépassent la distance du point de départ au point d'inversion d'une valeur seuil prédéterminée, et/ou
> - si aucune des mesures de distance du signal n'atteint une valeur seuil prédéterminée et si la personne testée n'atteint pas le point d'inversion, et/ou
> - lorsque le chronométrage atteint un temps maximal sans que le point final ait été atteint.

**9.** Procédé selon l'une quelconque des revendications 1 à 8, dans lequel le télémètre détermine la distance au moyen d'une mesure par ultrasons ou par laser.

**10.** Support de données comprenant un programme d'ordinateur qui, lorsqu'il est exécuté par un ordinateur, amène ce dernier à

> - recevoir des valeurs de mesure qui représentent le mouvement d'une personne le long d'un trajet,
> - créer un signal à partir des valeurs de mesure enregistrées au fil du temps,
> - déduire une fonction polynomiale comme modèle à partir de l'évolution du signal sur le trajet, **caractérisé en ce que** le programme d'ordinateur provoque en outre l'ordinateur lors de l'exécution,
> - comparer l'évolution du signal au modèle et déterminer une valeur de mesure pour la sécurité de marche est déterminée sur la base de cette comparaison.

**11.** Dispositif pour déterminer la sécurité de marche, comprenant

> - au moins un capteur pour mesurer le temps,
> - au moins un capteur pour enregistrer la position d'une personne qui se trouve sur un trajet prédéterminé,
> - une unité de traitement, dans lequel l'unité de traitement est conçue pour établir un signal à partir des valeurs de mesure enregistrées au fil du temps, pour déduire un modèle à partir de l'évolution du signal,

**caractérisé en ce que** l'unité d'évaluation est en outre conçue à cet effet,
comparer l'évolution du signal au modèle et déduire une valeur de mesure pour la sécurité de marche est déterminée sur la base de cette comparaison.

**12.** Dispositif selon la revendication 11, dans lequel le dispositif comprend :

> - une chaise avec une assise et un dossier,

>> - une unité de traitement reliée au fauteuil,
>> - un télémètre connecté à l'unité de traitement et relié au dossier, qui mesure en continu la distance entre une personne située devant une chaise et le dossier de la chaise, et
>> - un générateur de signal monté en aval de l'unité de traitement, qui est conçu pour émettre un signal de départ en présence d'un signal de déclenchement ou en cas de constatation qu'une personne est assise sur la chaise,
- une minuterie pouvant être activée par le signal de départ,

dans lequel l'unité de commande est conçue pour, après la fin du chronométrage, établir un signal à partir des valeurs de mesure enregistrées au fil du temps, et
- déduire une fonction polynomiale à partir de l'évolution du signal et
- comparer l'évolution du signal avec le modèle et fournir une valeur de mesure pour la sécurité de marche, en particulier le coefficient de détermination R2.

**Abbildung 1**

**Abbildung 2**

**Abbildung 3**

**Abbildung 4**

Abbildung 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- AT 518810
- JP 2016137226 A
- CN 103720476 A

**In der Beschreibung aufgeführte Nicht-Patentliteratur**

- **CHRISTINA STROHRMANN et al.** Monitoring motor capacity changes of children during rehabilitation using body-worn sensors. *JOURNAL OF NEUROENGINEERING AND REHABILITATION, BIOMED CENTRAL , LONDON,* 30. Juli 2013, vol. 10 (1), 1-16